(19) Europäisches Patentamt
European Patent Office
Office européen des brevets

(11) Veröffentlichungsnummer: **0 111 809**
**A1**

(12) # EUROPÄISCHE PATENTANMELDUNG

(21) Anmeldenummer: **83112196.7**

(22) Anmeldetag: **05.12.83**

(51) Int. Cl.³: **C 07 D 498/04**
C 07 D 265/28, A 61 K 31/535

(30) Priorität: **14.12.82 DE 3246148**
**11.06.83 DE 3321156**

(43) Veröffentlichungstag der Anmeldung:
**27.06.84 Patentblatt 84/26**

(84) Benannte Vertragsstaaten:
**AT BE CH DE FR GB IT LI LU NL SE**

(71) Anmelder: **Troponwerke GmbH & Co. KG**
**Berliner Strasse 156**
**D-5000 Köln 80(DE)**

(72) Erfinder: **Fruchtmann, Romanis**
**Konrad-Adenauer-Ufer 79**
**D-5000 Köln 1(DE)**

(72) Erfinder: **Horstmann, Harald, Dr.**
**Claudiusweg 19**
**D-5600 Wuppertal 1(DE)**

(72) Erfinder: **Mardin, Mithat, Dr.**
**Untere Bergerheide 15**
**D-5600 Wuppertal 1(DE)**

(72) Erfinder: **Opitz, Wolfgang, Dr.**
**Holzbachtalstrasse 60**
**D-5063 Overath(DE)**

(72) Erfinder: **Pelster, Bernhard, Dr.**
**Pleisufer 6a**
**D-5205 St. Augustin 1(DE)**

(72) Erfinder: **Raddatz, Siegfried, Dr.**
**Jakob-Böhme-Strasse 21**
**D-5000 Köln 80(DE)**

(74) Vertreter: **Jesse, Ralf-Rüdiger, Dr. et al,**
**Bayer AG Zentralbereich Patente Marken und Lizenzen**
**D-5090 Leverkusen 1 Bayerwerk(DE)**

(54) **Pyrazolo(4.3-b)(1.4)oxazine, Verfahren zu ihrer Herstellung und ihre Verwendung als Arzneimittel.**

(57) Die vorliegende Erfindung betrifft Pyrazolo[4.3-b][1.4] oxazine, Verfahren zu ihrer Herstellung, ihre Verwendung als Arzneimittel, insbesondere ihre Verwendung als Lipoxygenasehemmer, diese enthaltende Arzneimittel und deren Herstellung.

EP 0 111 809 A1

TROPONWERKE GmbH & Co. KG

5000 Köln 80
Je/bc/c

Pyrazolo[4.3-b][1.4]oxazine, Verfahren zu ihrer Herstellung und ihre Verwendung als Arzneimittel

Die vorliegende Erfindung betrifft Pyrazolo[4.3-b][1.4]-oxazine, ein Verfahren zu ihrer Herstellung, ihre Verwendung als Arzneimittel, insbesondere ihre Verwendung zur Behandlung von entzündlichen und/oder allergischen Prozessen und die Herstellung dieser Arzneimittel.

Bei den bisher üblichen Antiphlogistika handelt es sich überwiegend um Cyclooxygenasehemmer, die den Abbau der Arachidonsäure zu entzündungsfördernden Metaboliten hemmen. Diese Antiphlogistika haben folgenden Nachteil: Durch die Hemmung des Enzyms Cyclooxygenase kommt es zu einer weitreichenden Prostaglandinsynthetasehemmung, zu einer Stimulation des alternativen Lipoxygenasewegs und in der Folge zu einer proinflammatorischen bzw. asthmatischen Wirkung und zu Gastrotoxizität (vgl. K. Brune et al., J. Pharm. Pharmacol. 33, 127-128, 1981). Seit einiger Zeit ist man bemüht Stoffe zu finden, die in den alternativen Arachidonsäureabbau eingreifen, also als Lipoxygenasehemmer wirken. Es sind schon einige

wenige Lipoxygenasehemmer wie Nordihydroguaretsäure, 3-Amino-1-(3-trifluormethylphenyl)-pyrazolin, Phenidon und 5.8.11.14-Eikosatetrainsäure bekannt. Ihre Wirkung ist jedoch recht schwach - meist ist ihre Hauptwirkung eine Cyclooxygenasehemmwirkung - und einige, z.B. 3-Amino-1-(trifluormethylphenyl)-pyrazolin zeigen bei oraler Verabreichung toxische Nebenwirkungen. Es besteht daher ein Bedarf an oral wirksamen Verbindungen, die solche unerwünschten Nebenwirkungen nicht besitzen.

Überraschenderweise sind die neuen, erfindungsgemäßen Pyrazolo[4.3-b][1.4]oxazine potente Hemmstoffe der Lipoxygenase. Zum Teil hemmen sie die Lipoxygenase schon in solchen Konzentrationen, bei denen die Cyclooxygenase noch nicht beeinflußt wird.

Die neuen, erfindungsgemäßen Verbindungen stimulieren auch die Synthese von Prostacyclin in arteriellen Gefäßen in vitro. Die erfindungsgemäßen Verbindungen stimulieren auch an Kaninchenaortenstreifen die Prostacyclinsynthese.

Die neuen, erfindungsgemäßen Verbindungen besitzen eine antiphlogistische Wirkung im durch Carrageenan induzierten Ödemmodell, wenn sie systemisch, besonders oral und lokal, besonders cutan, verabreicht werden.

Die neuen, erfindungsgemäßen Verbindungen besitzen ferner eine antimetastatische Wirkung.

Die neuen, erfindungsgemäßen Verbindungen wirken analgetisch; sie sind wirksam im Hyperalgesietest nach Randall-Selitto.

Die neuen, erfindungsgemäßen, lipoxygenasehemmenden Pyrazolo-oxazine entsprechend der Formel (I) sind sowohl einzeln entsprechend der Formel Ia oder Ib wie auch als Gemisch von Ia und Ib als Arzneimittel bei der Behandlung von entzündlichen und allergischen Prozessen einsetzbar. Sie können insbesondere als Antiphlogistika, Antirheumatika, Antiatherosklerotika, Analgetika, Antithrombotika, Antiarthrotika, Antiasthmatika, Antiallergika, Antipsoriatika, Antimetastatika und Gastroprotektiva Verwendung finden.

Die neuen, erfindungsgemäßen Pyrazolo[4.3-b][1.4]oxazine entsprechen der allgemeinen Formel I in ihren isomeren Formen Ia und I b:

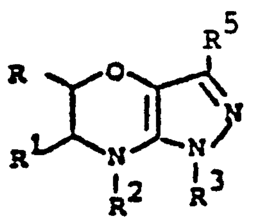

Ia

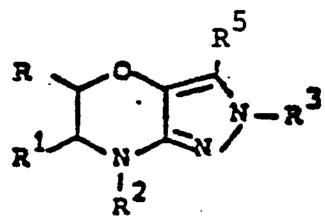

Ib

I

jeweils einzeln entsprechend der Formel Ia oder Ib als auch im Gemisch der Formel Ia und Ib, hier als I bezeichnet, wobei

R und $R^1$ gleich oder verschieden sein können und für Wasserstoff, Alkyl mit 1 bis 18 C-Atomen und Phenyl, gegebenenfalls substituiert mit bis zu fünf gleichen oder verschiedenen Substituenten aus der Gruppe Alkyl mit 1 bis 6 C-Atomen, Cycloalkyl mit 3 bis 6 C-Atomen, Aralkyl mit 7 bis 22 C-Atomen, Aryl mit 6 bis 14 C-Atomen, Mono- und Dialkylamino mit 1 bis 12 C-Atomen, Arylamino mit 6 bis 10 C-Atomen, Aryloxy mit 6 bis 10 C-Atomen, Alkylthio mit 1 bis 12 C-Atomen, Arylthio mit 6 bis 10 C-Atomen, Aralkoxy mit 7 bis 22 C-Atomen, Aralkylamino mit 7 bis 16 C-Atomen, Carboxy, Carbalkoxy mit 2 bis 13 C-Atomen, Carbamoyl, Mono- und Dialkylcarbamoyl mit 1 bis 12 C-Atomen, Alkylsulfonyl mit 1 bis 12 C-Atomen, Arylsulfonyl mit 6 bis 14 C-Atomen, Halogenalkyl mit bis zu 12 C-Atomen, gegebenenfalls ein- oder mehrfach substituiert mit F, Cl, Br oder/und J, Hydroxyalkyl mit 1 bis 12 C-Atomen, Aminoalkyl mit 1 bis 12 C-Atomen, Alkoxycarbonylaminoalkyl mit 3 bis 13 C-Atomen, Halogenalkoxy mit bis zu 12 C-Atomen, gegebenenfalls einfach oder mehrfach substituiert mit F, Cl, Br oder/und J, Halogen wie F, Cl, Br und J, Amino, Acylamino mit 2 bis 7 C-Atomen, Carbamoylamino, Sulfonylamino, Hydroxy, Acyloxy mit 2 bis 7 C-Atomen, Sulfonamido, Nitro, Formyl, Alkylcarbonyl mit 2 bis 13 C-Atomen, Arylcarbonyl mit 7 bis 15 C-Atomen, Methylencarboxy, Methylencarboxyalkyl mit 3 bis 8 C-Atomen, Ethylencarboxyalkyl mit 4 bis 9 C-Atomen oder Propylencarboxyalkyl mit 5 bis 10 C-Atomen stehen,

$R^2$ und $R^3$ gleich oder verschieden sein können und für Wasserstoff, Alkyl mit 1 bis 18 C-Atomen stehen, wobei der Alkylrest durch Sauerstoff, Schwefel oder Stickstoff ein- oder mehrfach unterbrochen sein kann, Alkenyl oder Alkinyl mit 2 bis 12 C-Atomen, Arylalkyl mit 7 bis 12 C-Atomen, wobei die Alkenyl-, Alkinyl- und Aralkylreste ebenfalls durch Sauerstoff, Schwefel oder Stickstoff ein- oder mehrfach unterbrochen sein können, für Aryl stehen mit 6 bis 14 C-Atomen, 5- bis 6-gliedriges Heteroaryl mit bis zu 4 Heteroatomen aus der Reihe N, O und S, 5- bis 6-gliedriges Heteroarylalkyl mit bis zu 4 Heteroatomen aus der Reihe N, O und S mit 1 bis 12 C-Atomen in der Alkylkette, Alkylcarbonyl mit 2 bis 13 C-Atomen, Arylcarbonyl mit 7 bis 15 C-Atomen, ggf. mit bis zu 5 gleichen oder verschiedenen Substituenten aus der Gruppe Alkyl mit 1 bis 18 C-Atomen, Cycloalkyl mit 3 bis 6 C-Atomen, Aralkyl mit 7 bis 22 C-Atomen, Aryl mit 6 bis 14 C-Atomen, Mono- und Dialkylamino mit 1 bis 12 C-Atomen, Arylamino mit 6 bis 10 C-Atomen, Aryloxy mit 6 bis 10 C-Atomen, Alkylthio mit 1 bis 12 C-Atomen, Arylthio mit 6 bis 10 C-Atomen, Aralkoxy mit 7 bis 22 C-Atomen, Aralkylamino mit 7 bis 16 C-Atomen, Carboxy, Carbalkoxy mit 2 bis 13 C-Atomen, Carbamoyl, Mono- und Dialkylcarbamoyl mit 1 bis 12 C-Atomen, Alkylsulfonyl mit 1 bis 12 C-Atomen, Arylsulfonyl mit 6 bis 14 C-Atomen, Halogenalkyl mit

bis zu 12 C-Atomen, ggf. einfach oder mehrfach substituiert mit F, Cl, Br oder/und J, Hydroxyalkyl mit 1 bis 12 C-Atomen, Aminoalkyl mit 1 bis 12 C-Atomen, Alkoxycarbonylaminoalkyl mit 3 bis 13 C-Atomen, Halogenalkoxy mit bis zu 12 C-Atomen, ggf. einfach oder mehrfach substituiert mit F, Cl, Br oder/und J, Halogen wie F, Cl, Br oder/und J, Amino, Acylamino mit 2 bis 7 C-Atomen, Sulfonamido, Nitro, Formyl, Methylencarboxy, Methylencarboxyalkyl mit 3 bis 8 C-Atomen, Ethylencarboxyalkyl mit 4 bis 9 C-Atomen oder Propylencarboxyalkyl und für 5- bis 6-gliedriges Heteroarylcarbonyl mit bis zu 4 Heteroatomen aus der Reihe N, O und S und

$R^5$ für Wasserstoff, für einen unverzweigten, verzweigten oder cyclischen Alkylrest, jeweils mit 1 bis 6 C-Atomen, steht, welcher ggf. durch Hydroxy, Carboxy, Formyl, Acetyl, Alkoxy, Alkylthio, Halogen, Nitro, substituierte oder freie Aminogruppen substituiert sein kann, für einen aromatischen oder heteroaromatischen Rest mit bis zu 4 Heteroatomen aus der Reihe N, O und S steht, der ggf. durch Alkyl, Alkoxy, Alkylthio oder Halogen substituiert sein kann oder für einen in gleicher Weise substituierten Aralkyl- oder Heteroaralkylrest steht.

Bevorzugt sind Pyrazolo/4.3-b7/1.47oxazine der allgemeinen Formel (I), in ihren isomeren Formen entsprechend den Formeln Ia und Ib, in welcher jeweils

$R^1$ und $R^2$ gleich oder verschieden sein können und für Phenyl, ggf. mit bis zu 5 gleichen oder verschiedenen Substituenten aus der Gruppe Alkyl mit 1 bis 6 C-Atomen, Aralkyl mit 7 bis 15 C-Atomen, Phenyl oder Naphthyl, Mono- und Dialkylamino mit 1 bis 8 C-Atomen, Arylamino mit 6 bis 10 C-Atomen, Aryloxy mit 6 bis 10 C-Atomen, Alkylthio mit 1 bis 12 C-Atomen, Aralkoxy mit 7 bis 16 C-Atomen, Aralkylamino mit 7 bis 16 C-Atomen, Carboxy, Carbalkoxy mit 2 bis 7 C-Atomen, Carbamoyl, Mono- und Dialkylcarbamoyl mit 1 bis 6 C-Atomen, Alkylsulfonyl mit 1 bis 12 C-Atomen, Arylsulfonyl mit 6 bis 14 C-Atomen, Halogenalkyl mit bis zu 6 C-Atomen, ggf. einfach oder mehrfach substituiert mit F, Cl oder/und Br, Hydroxyalkyl mit 1 bis 6 C-Atomen, Aminoalkyl mit 1 bis 6 C-Atomen, Alkoxycarbonylaminoalkoxy mit 3 bis 9 C-Atomen, Halogenalkoxy mit bis zu 6 C-Atomen, ggf. einfach oder mehrfach substituiert mit F, Cl oder/und Br, Halogen wie F, Cl und Br, Amino, Acylamino mit 2 bis 7 C-Atomen, Carbonylamino, Sulfonylamino, Hydroxy, Acyloxy mit 2 bis 7 C-Atomen, Sulfonamido, Nitro, Formyl, Alkylcarbonyl mit 2 bis 7 C-Atomen, Arylcarbonyl mit 7 bis 11 C-Atomen, Methylencarboxy, Methylencarboxyalkyl mit 3 bis 8 C-Atomen, Ethylencarboxyalkyl mit 4 bis 9 C-Atomen und Propylencarboxyalkyl substituiert stehen,

$R^2$ und $R^3$ gleich oder verschieden sein können und für Wasserstoff, Alkyl mit 1 bis 12 C-Atomen stehen, wobei der Alkylrest durch Sauerstoff, Schwefel oder Stickstoff ein- oder mehrfach unterbrochen sein kann, Alkenyl oder Alkinyl mit 2 bis 6 C-Atomen, Aralkyl mit 7 bis 12 C-Atomen, wobei die Alkenyl-, Alkinyl- und Aralkylreste ebenfalls durch Sauerstoff, Schwefel oder Stickstoff ein- oder mehrfach unterbrochen sein können, für Phenyl und Naphthyl stehen, 5- bis 6-gliedriges Heteroaryl mit bis zu drei Heteroatomen aus der Reihe N, O und S, 5- bis 6-gliedriges Heteroarylalkyl mit bis zu drei Heteroatomen aus der Reihe N, O und S mit 1 bis 6 C-Atomen in der Alkylkette, Alkylcarbonyl mit 2 bis 13 C-Atomen, Arylcarbonyl mit 7 bis 11 C-Atomen, ggf. mit bis zu 5 gleichen oder verschiedenen Substituenten aus der Gruppe Alkyl mit 1 bis 6 C-Atomen, Cycloalkyl mit 3 bis 6 C-Atomen, Aralkyl mit 7 bis 11 C-Atomen, Arylamino mit 6 bis 10 C-Atomen, Aryloxy mit 6 bis 10 C-Atomen, Alkylthio mit 1 bis 6 C-Atomen, Arylthio mit 6 bis 10 C-Atomen, Aralkoxy mit 7 bis 12 C-Atomen, Aralkylamino mit 7 bis 12 C-Atomen, Carboxy, Carbalkoxy mit 2 bis 7 C-Atomen, Carbamoyl, Mono- und Dialkylcarbamoyl mit 1 bis 6 C-Atomen, Alkylcarbonyl mit 1 bis 12 C-Atomen, Alkylsulfonyl mit 1 bis 12 C-Atomen, Arylsulfonyl mit 6 bis 14 C-Atomen, Halogenalkyl mit bis zu 6 C-Atomen, ggf. einfach oder mehrfach substituiert mit F, Cl oder/ und Br, Hydroxyalkyl mit 1 bis 6 C-Atomen,

Aminoalkyl mit 1 bis 6 C-Atomen, Alkoxycarbonylaminoalkoxy mit 3 bis 9 C-Atomen, Halogenalkoxy mit bis zu 6 C-Atomen, ggf. einfach oder mehrfach substituiert mit F, Cl oder/und Br, Halogen wie F, Cl oder/und Br, Amino, Acylamino mit 2 bis 7 C-Atomen, Carbamoylamino, Sulfonylamino, Hydroxy, Acyloxy mit 2 bis 7 C-Atomen, Sulfonamido, Nitro, Formyl, Alkylcarbonyl mit 2 bis 13 C-Atomen, Arylcarbonyl mit 7 bis 15 C-Atomen, Methylencarboxy, Methylencarboxyalkyl mit 3 bis 8 C-Atomen, Ethylencarboxyalkyl mit 4 bis 9 C-Atomen oder Propylencarboxyalkyl mit 5 bis 10 C-Atomen und 5- bis 6-gliedriges Heteroarylcarbonyl mit bis zu 3 Heteroatomen aus der Reihe N, O und S substituiert sein kann und

$R^5$ für Wasserstoff, für einen unverzweigten, verzweigten oder cyclischen Alkylrest steht, jeweils mit 1 bis 6 C-Atomen, welcher ggf. durch Hydroxy, Carboxy, Formyl, Acetyl, Alkoxy, Alkylthio, Halogen, Nitro, substituierte oder freie Aminogruppen substituiert sein kann, für einen aromatischen oder heteroaromatischen Rest mit bis zu 4 Heteroatomen aus der Reihe N, O und S steht, der ggf. durch Alkyl, Alkoxy, Alkylthio oder Halogen substituiert sein kann oder für einen in gleicher Weise substituierten Aralkyl- oder Heteroaralkylrest steht.

Besonders bevorzugt sind die neuen, erfindungsgemäßen Verbindungen der allgemeinen Formeln Ia und Ib, in welcher

R und $R^1$ gleich oder verschieden sein können und für Wasserstoff, Alkyl mit 1 bis 6 C-Atomen, ggf. mit bis zu 5 Fluoratomen substituiert und/oder Phenyl stehen, ggf. mit bis zu 5 Substituenten substituiert aus der Gruppe Alkyl mit 1 bis 4 C-Atomen je Alkylgruppe, Alkyloxy mit 1 bis 3 C-Atomen, Aryloxy mit 6 C-Atomen und mit F, Cl, Br, $NO_2$, $NH_2$, NH-niederes Alkyl, N(niederes Alkyl)$_2$, Alkylthio mit 1 bis 6 C-Atomen, Alkylsulfonyl mit 1 bis 6 C-Atomen, mit Aminocarbonyl und Aminosulfonyl,

$R^2$ und $R^3$ gleich oder verschieden sind und für Wasserstoff, Alkyl mit bis zu 6 C-Atomen, Arylalkyl mit 3 bis 13 C-Atomen, ggf. substituiert mit bis zu 5 F, Cl oder Br, Aryloxyalkyl mit 7 bis 16 C-Atomen, Formyl, Alkylcarbonyl mit 2 bis 13 C-Atomen, Carboxyalkyl mit 2 bis 7 C-Atomen und für Arylalkyloxycarbonyl mit 8 bis 16 C-Atomen stehen und

$R^5$ für Wasserstoff, für einen unverzweigten oder verzweigten Alkylrest mit 1 bis 6 C-Atomen steht, der ggf. durch Alkoxy substituiert sein kann, oder für einen Aryl- oder Heteroarylrest steht; bevorzugt sind Phenyl- oder Pyridylreste, die gegebenenfalls durch Fluor, Chlor, Alkyl oder Alkoxy substituiert sind.

Die neuen, erfindungsgemäßen Verbindungen der allgemeinen Formeln Ia und Ib lassen sich herstellen, indem man

a) die 1,4-Oxazine der allgemeinen Formel II, in welcher R, $R^1$ und $R^2$ die obengenannte Bedeutung haben, mit einem N,N-Dialkylamid der allgemeinen Formel VI

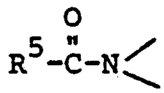

VI

(vgl. Chem. Ber. 92, 837, 1959 und J. Het. Chem. 19, 1493, 1982) mit der für $R^5$ oben angegebenen Bedeutung und Phosphoroxychlorid im Sinne einer Vilsmeier-Haack-Reaktion umsetzt und die dabei entstehenden Salze der allgemeinen Formel III mit Hydrazinen der allgemeinen Formel IV umsetzt:

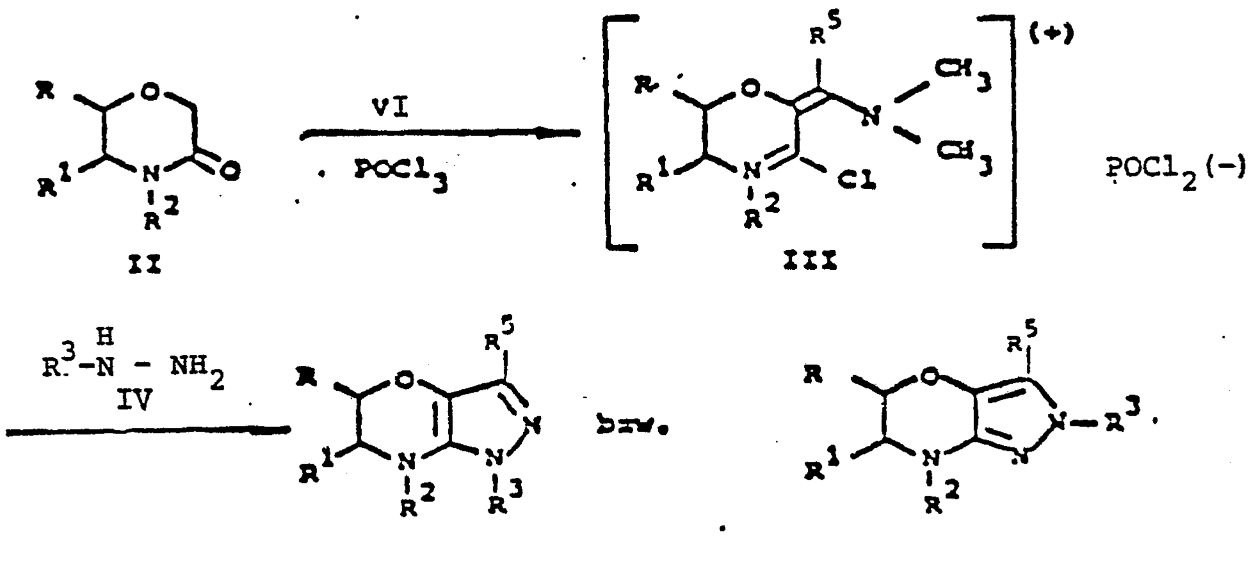


II, III, Ia bzw. Ib

Die Umsetzung der Salze der allgemeinen Formel III mit den Hydrazinen der allgemeinen Formel IV geschieht vorzugsweise in polaren Lösungsmitteln, z.B. in Alkanolen, ohne Katalysator oder auch in Gegenwart von organischen Basen, wie z.B. Triethylamin, bei Temperaturen zwischen -20 und +100°C. Die Reste R, $R^1$, $R^2$, $R^3$ und $R^5$ haben die vorstehend genannte Bedeutung.

Bevorzugt werden die Salze der allgemeinen Formel III mit den Hydrazinen der allgemeinen Formel IV in Ethanol bei Raumtemperatur bis Siedetemperatur, insbesondere bei 80°C, umgesetzt, um die neuen, erfindungsgemäßen Verbindungen zu erhalten,

oder

b) die Oxazine der allgemeinen Formel II, in welcher R, $R^1$ und $R^2$ die obengenannte Bedeutung haben, mit Carbonsäureestern der allgemeinen Formel VII

$$R^5-\overset{O}{\overset{\|}{C}}-O-R^6 \qquad VII$$

worin $R^5$ die oben angegebene Bedeutung (exklusive Wasserstoff) hat, und $R^6$ für einen Niedrigalkylrest, bevorzugt Methyl oder Ethyl, steht, in Dimethylsulfoxid oder dem Carbonsäureester selbst als Lösungsmittel mit Natriumhydrid zu den 2-Acyl-1,4-oxazinonen VIII umsetzt (vgl. Arch. Pharm. 312, 302, 1979). Die 2-Acyl-1,4-oxazinone VIII werden weiter zu den Thionen IX umgesetzt (mit $P_4S_{10}$,

vgl. u.a. Tetrahedron 25, 517, 1969 oder mit Lawesson's Reagens, vgl. u.a. Org. Prep. Proc. Int. 12, 203, 1980). Deren Kondensation mit den Hydrazinen IV liefert die erfindungsgemäßen Verbindungen der allgemeinen Formeln Ia und Ib.

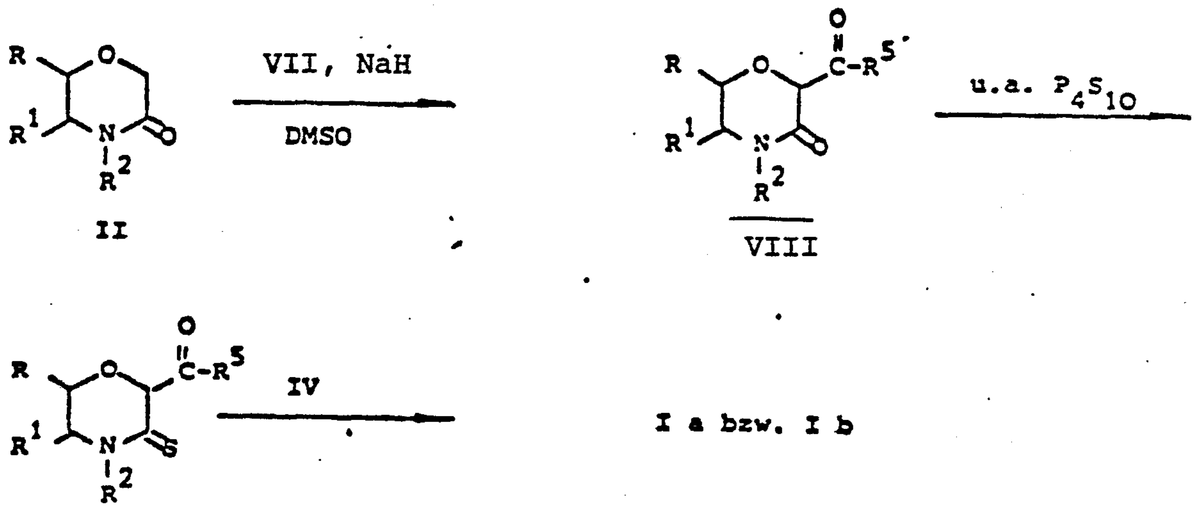


II

VIII

I a bzw. I b

Die neuen erfindungsgemäßen Verbindungen der allgemeinen isomeren Formen gemäß Formeln Ia und Ib, in denen $R^3$ für Alkylcarbonyl und/oder Arylalkylcarbonyl mit der oben angegebenen Bedeutung stehen, lassen sich herstellen, indem man die neuen, erfindungsgemäßen Verbindungen der allgemeinen Formeln Ia und Ib, in der $R^2$ und/oder $R^3$ für Wasserstoff stehen, mit Acylierungsmitteln, z.B. Säurehalogeniden oder Säureanhydriden der allgemeinen Formel V, in der $R^4$ die gleiche Bedeutung wie $R^2$ oder $R^3$ hat, in Gegenwart einer Hilfsbase, z.B. Pyridin, umsetzt.

$$R^4-\overset{O}{\overset{\|}{C}}-Y \qquad Y = \text{Imidazol}, -O-CO-R_4, \text{Cl, Br, J}$$

V

Die für die Durchführung der Erfindung geeigneten Verbindungen der Formel II sind bekannt oder lassen sich nach bekannten Methoden herstellen (vgl. Shah et al., Indian Journal of Chemistry 7, 1006, 1969 und 10, 820, 1972).

Die für die Durchführung der Erfindung geeigneten Hydrazine der allgemeinen Formel IV sind bekannt oder lassen sich nach bekannten Methoden herstellen.

Besonders bevorzugte Beispiele der neuen, erfindungsgemäßen Verbindungen sind (vgl. Tabelle 1):

3,7-Dimethyl-5-phenylpyrazolo[4.3-b][1.4]oxazin
3,7-Dimethyl-5,6-diphenylpyrazolo[4.3-b][1.4]oxazin
3,7-Dimethyl-5,6-di-(4-chlorphenyl)pyrazolo[4.3-b][1.4]oxazin
3,7-Dimethyl-5,6-di-(4-methoxyphenyl)pyrazolo[4.3-b][1.4]oxazin
1-Acetyl-3,7-dimethyl-5,6-diphenylpyrazolo[4.3-b][1.4]oxazin
7-Methyl-3-propyl-5,6-diphenylpyrazolo[4.3-b][1.4]oxazin
7-Methyl-3-isopropyl-5,6-diphenylpyrazolo[4.3-b][1.4]oxazin
3-(4-Chlorphenyl)-7-methyl-5,6-diphenylpyrazolo[4.3-b][1.4]oxazin
3-(3-Pyridyl)-7-methyl-5,6-diphenylpyrazol[4.3-b][1.4]oxazin

7-Methylpyrazolo[4.3-b][1.4]oxazin
1.7-bzw. 2.7-Dimethylpyrazolo[4.3-b][1.4]oxazin
2-(3.4-Dichlorbenzyl)-7-methylpyrazolo[4.3-b][1.4]oxazin
1-bzw. 2-(2-Phenoxyethyl)-7-methyl[4.3-b][1.4]oxazin
1-bzw. 2-(ß-Naphthoxyethyl)-7-methylpyrazolo[4.3-b][1.4]-oxazin
5-Phenylpyrazolo[4.3-b][1.4]oxazin
2-Methyl-5-phenylpyrazolo[4.3-b][1.4]oxazin
7-Methyl-5-phenylpyrazolo[4.3-b][1.4]oxazin
1.7-bzw. 2.7-Dimethyl-5-phenylpyrazolo[4.3-b][1.4]oxazin
2-Formyl-7-methyl-5-phenylpyrazolo[4.3-b][1.4]oxazin
2-Acetyl-7-methyl-5-phenylpyrazolo[4.3-b][1.4]oxazin
2-Benzyloxycarbonyl-7-methyl-5-phenylpyrazolo[4.3-b][1.4]-oxazin
7-Methyl-5.6-diphenylpyrazolo[4.3-b][1.4]oxazin
7-Methyl-5.6-di-(4-methoxyphenyl)-pyrazolo[4.3-b][1.4]-oxazin
1.7- bzw. 2.7-Dimethyl-5.6-diphenylpyrazolo[4.3-b][1.4]-oxazin
1.7-bzw. 2.7-Dimethyl-5.6-di-(4-chlorphenyl)-pyrazolo-[4.3-b][1.4]oxazin
2-Aceryl-7-methyl-5.6-diphenylpyrazolo[4.3-b][1.4]oxazin
2-Carboxymethyl-7-methyl-5.6-diphenylpyrazolo [4.3-b]-[1.4]oxazin.

Der Nachweis der lipoxygenasehemmenden Eigenschaften der neuen, erfindungsgemäßen Verbindungen erfolgt analog der Methode von Bailey et al., J. Biol. Chem. <u>255</u>, 5996, 1980 und nach Blackwell and Flower, Prostaglandins <u>16</u>, 417, 1978. Bei dieser Testmethode wird der Metabolismus radioaktiv markierter Arachidonsäure an gewaschenen Humanthrombozyten untersucht. Bei

diesem in-vitro-Test werden die radioaktiv markierten Metaboliten aus dem Reaktionsansatz extrahiert und dünnschichtchromatographisch getrennt. Das Autoradiogramm wird mit Dünnschichtscanner ausgewertet. Bei diesen Testbedingungen werden die markierten Metaboliten von der nicht umgesetzten Arachidonsäure getrennt und sind anschließend auszuwerten. Die Verteilung der Radioaktivität auf die während der Metabolisierung gebildeten Cyclooxygenaseprodukte Thromboxan $B_2$ ($TXB_2$) und 12-Hydroxy-5.8.10-heptadekatriensäure (HHT) bzw. das Lipoxygenaseprodukt 12-Hydroxy-5.8.11.14-eikosatetraensäure (HETE) unter dem Einfluß der Inhibitoren stellt ein Maß für die Hemmung der Enzyme dar.

Die Lipoxygenasehemmung der erfindungsgemäßen Verbindungen läßt sich an der Hemmung der HETE-Synthesen messen. Es zeigt sich, daß die Synthese von $TXB_2$ und von HHT unbeeinflußt bleibt, während der Arachidonsäureumsatz abnimmt. Wie aus der nachfolgenden Tabelle ersichtlich ist, bewirken die erfindungsgemäßen Verbindungen eine signifikante Hemmung der Plättchenlipoxygenase (HETE-Synthese).

Analog dem o.a. Test lassen sich die lipoxygenasehemmenden Eigenschaften der neuen, erfindungsgemäßen Verbindungen auch an Leukozyten nachweisen. Die polymorphkernigen Leukozyten des Menschen und des Kaninchens metabolisieren die Arachidonsäure zu 5-Hydroxy-5.8.11.14-eikosatetraensäure (5-HETE) und Leukotrien $B_4$ (5S, 12R-Dihydroxy-6 cis, 8.10 trans, 14 ciseikosatetraensäure). Die Hemmung der

Freisetzung von 5-HETE und Leukotrien $B_4$ aus den Leukozyten stellt ein Maß für den lipoxygenasehemmenden Effekt der neuen, erfindungsgemäßen Verbindungen dar (vgl. Tabelle A).

Der Test mit den Humanleukozyten wird nach Borgeat and Samuelsson (J.Biol. Chem. 254, 2643, 1979 und Proc. Natl. Acad. Sci. U.S.A. 76, 2148, 1979), mit Kaninchenleukozyten nach Walker and Parish (Intern. Archs. Allergy Appl. Immun. 66, 83, 1981) durchgeführt.

Der Nachweis der Prostacyclin-stimulierenden Wirkung erfolgte durch Bestimmung der Freisetzung von Prostacyclin nach einstündiger Inkubation von Kaninchenaortenstreifen mit den erfindungsgemäßen Verbindungen (analog nach Moncada et al., Lancet 1977, I, 18) und anschließender radioimmunologischer Bestimmung des stabilen Prostacyclinmetaboliten 6-Keto-PGF $1\alpha$ (B.M. Peskar et al., FEBS Letters 121, 25, 1980).

Die erfindungsgemäßen Verbindungen sind auch in-vivo wirksam. Diese Wirkung wird durch die Messung der Hemmung der Leukozytenmigration nach an sich bekannten Methoden nachgewiesen (vgl. Higgs et al., Biochemical Pharmacology 28, 1959, (1979) und Eur. J. Pharmacol. 66,81 (1981)). Die erfindungsgemäßen Verbindungen reduzieren auch die Ödembildung im Carrageenan-Entzündungsmodell, (vgl. Tabelle C) und hemmen die Metastasierung von B-16 Melanom (nach Honn, et.al. Science 212, 1981).

Die erfindungsgemäßen Verbindungen sind auch in vivo wirksam im Hyperalgesie-Test nach Randall-Selitto, vgl. Tabelle B (Randall, L.O., Selitto, J., Arch. Int. Pharmakodyn, 111, 209-219, 1957).

Ein weiterer Gegenstand der vorliegenden Erfindung ist daher noch die Verwendung einer oder mehrerer der Verbindungen gemäß allgemeiner Formel I bei der Bekämpfung von Krankheiten, bevorzugt von entzündlichen Prozessen, insbesondere als Lipoxygenasehemmer und/oder als Analgetika als Gefäßwand- und/oder Cytoprotektiva, besonders als Antithrombotikum, Antimetastatikum, Antiallergikum, Antiasthmatikum, Ulcus-Präventivum und/oder Anurektikum.

<u>Tabelle A</u>

| Nr. | Struktur | Minimale Konzentration (mol/l) für die Hemmung der Lipoxygenase in PMN-Leukozyten (5-HETE) |
|---|---|---|
| 1 | 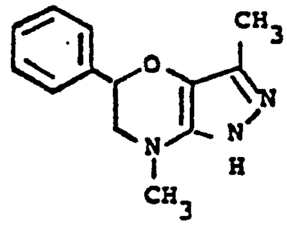 | > $10^{-5}$ |
| 2 | 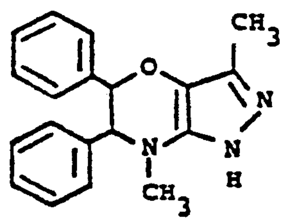 | 5 . $10^{-6}$ |
| 3 | 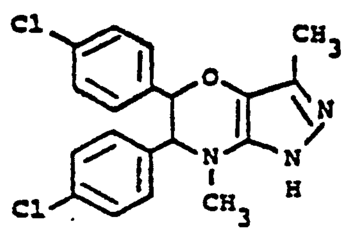 | 5 . $10^{-6}$ |
| 4 | 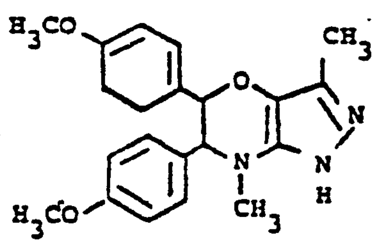 . HCl | $10^{-5}$ |
| 5 | 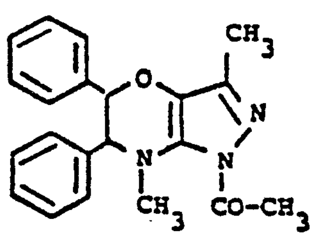 | > $10^{-5}$ |

Tabelle A (Fortsetzung)

| Nr. | Struktur | Minimale Konzentration (mol/l) für die Hemmung der Lipoxygenase in PMN-Leukozyten (5-HETE) |
|---|---|---|
| 6 | 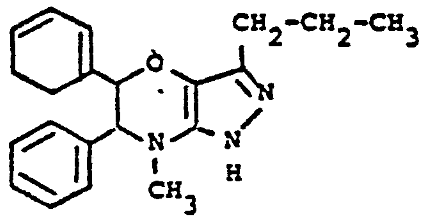 | $5 \cdot 10^{-6}$ |
| 7 | 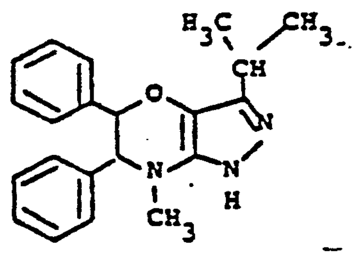 | $5 \cdot 10^{-6}$ |
| 8 | 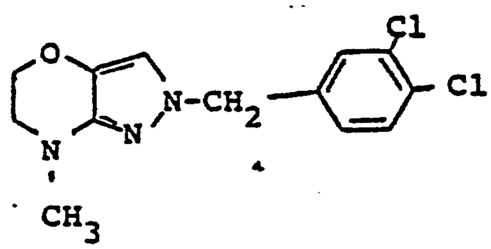 | $10^{-5}$ |
| 9 | 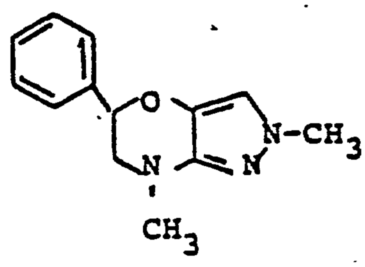 | $10^{-5}$ |
| 10 | 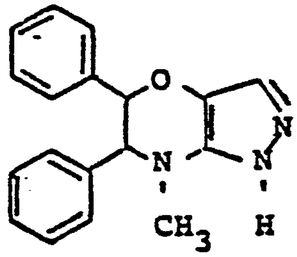 | $5.10^{-6}$ |

Tabelle B

Hemmung der Hyperalgesie nach Randall-Selitto nach oraler Gabe

| Nr. | Verbindung | $DE_{50}$ [mg/kg], oral |
|---|---|---|
| 1 | 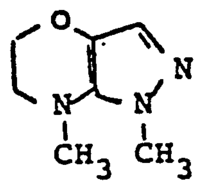 + 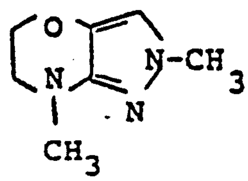<br>8 : 1 im Gemisch | 19,7 |
| 2 | 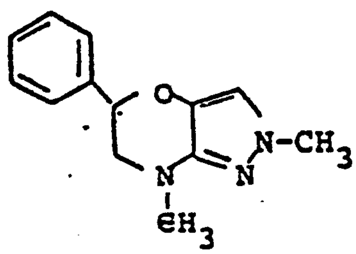 | 0,80 |
| 3 | 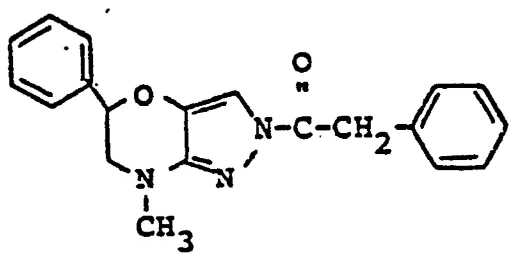 | 1,23 |
| 4 | 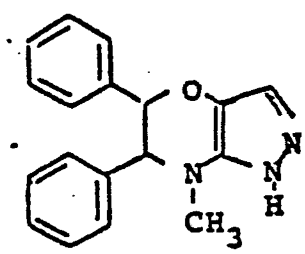 | 7,7 |

Tabelle C

Hemmung der Carrageenanödembildung nach oraler Gabe

| Nr. | Verbindung | $DE_{50}$ [mg/kg], oral |
|---|---|---|
| 1 | 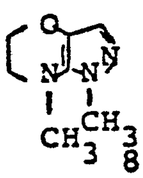 : 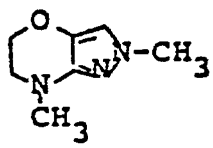 8 : 1 im Gemisch | 13,3 |
| 2 | 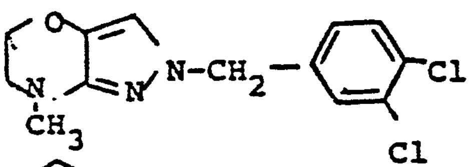 | 10,3 |
| 3 | 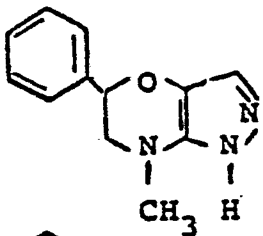 | 12,7 |
| 4 | 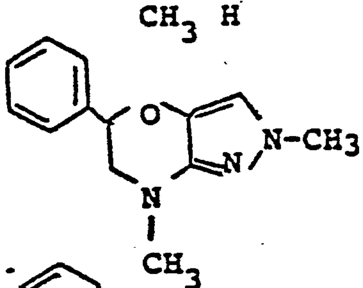 | 11,3 |
| 5 | 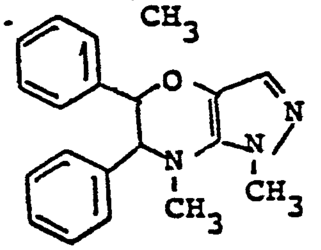 | 14,2 |
| 6 | 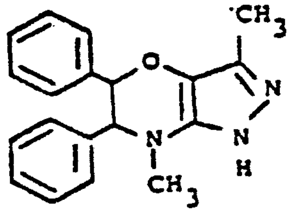 | 2,5 |

Die neuen Wirkstoffe können in bekannter Weise in die üblichen Formulierungen wie Tabletten, Kapseln, Dragees, Pillen, Granulate, Aerosole, Sirupe, Emulsionen, Suspensionen und Lösungen, unter Verwendung inerter, nichttoxischer pharmazeutisch geeigneter Trägerstoffe oder Lösungsmittel überführt werden. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Falle der Benutzung von Wasser als Verdünnungsmittel gegebenenfalls organische Lösungsmittel als Hilfslösungsmittel verwendet werden können.

Als Hilfsstoffe seien beispielhaft aufgeführt:

Wasser, nicht-toxische organische Lösungsmittel, wie Paraffine (z.B. Erdölfraktionen), pflanzliche Öle, (z.B. Erdnuß-/Sesamöl), Alkohole (z.B. Ethylalkohol, Glycerin), Glykole (z.B. Propylenglykol, Polyethylenglykol), N-Alkylpyrrolidone, feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Roh-, Milch- und Traubenzucker), Emulgiermittel, wie nichtionogene anionische Emulgatoren (z.B. Polyoxyethylen-Fettsäure-Ester, Polyoxyethylen-Fettalkohol-Ether,

Alkylsulfonate und Arylsulfonate), Dispergiermittel (z.B. Lignin, Sulfitablaugen, Methylcellulose, Stärke und Polyvinylpyrrolidon) und Gleitmittel (vorzugsweise Magnesiumstearat, Talkum, Stearinsäure und Natriumlaurylsulfat).

Die Applikation erfolgt vorzugsweise oral oder parenteral, vorzugsweise cutan. Im Falle der oralen Anwendung können Tabletten selbstverständlich außer den genannten Trägerstoffen auch Zusätze wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder gefärbten bzw. farbgebenden Stoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,05 bis 5 mg/kg Körpergewicht pro Tag zur Erzielung wirksamer Ergebnisse zu verabreichen, und bei oraler Applikation beträgt die Dosierung etwa 0,05 bis 100 mg/kg, vorzugsweise 0,1 bis 10 mg/kg Körpergewicht pro Tag.

Trotzdem kann es gegebenenfalls erforderlich sein, von den genannten Mengen abzuweichen, und zwar in Abhängigkeit vom Körpergewicht bzw. der Art des Applikationsweges, aber auch aufgrund der Art und deren individuellen Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Die obigen Ausführungen gelten für die Applikation sowohl in der Humanals auch in der Tiermedizin in sinngemäß gleicher Weise.

Die folgenden Beispiele sollen die Erfindung näher erläutern:

Beispiel 1

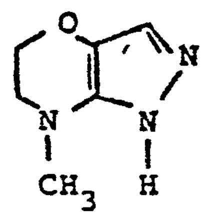

7-Methylpyrazolo[4.3-b][1.4]oxazin

Bei 0°C tropfte man in eine Lösung von 12,2 ml (0,133 Mol) Phosphoroxychlorid in 32,5 ml abs. 1,2-Dichlorethan 12,4 ml (0,16 Mol) Dimethylformamid zu. Man ließ 10 Minuten bei dieser Temperatur rühren. Dazu tropfte man unter Rühren 15,0 g ( 0,13 Mol) N-Methylmorpholon-3 in 32,5 ml abs. 1,2-Dichlorethan, so daß 5°C nicht überschritten wurden. Die entstandene, kräftig rot gefärbte Lösung rührte man noch 30 Minuten bei 0°C, drei Stunden bei Raumtemperatur und eine Stunde bei 45°C. Anschließend destillierte man das Lösungsmittel bei maximal 40°C Badtemperatur i.V. ab (Rotavapor). Den Rückstand nahm man in 170 ml Ethanol auf und tropfte unter Rühren zur auf 0°C abgekühlten, klaren, roten Lösung 50 ml (1,03 Mol) Hydrazinhydrat. Beim Zutropfen fiel sofort ein klumpiger, roter Niederschlag aus (Hydrazon). Man rührte noch eine Stunde bei 5°C und zwei Stunden bei Raumtemperatur, wobei sich der Niederschlag nach und nach auflöste und eine klare, tiefrot gefärbte

Lösung entstand. Man dampfte den Alkohol i.V. ab (Rotavapor) und reinigte den Rückstand säulenchromatographisch (Kieselgel 60, Merck; Laufmittel: Dichlormethan/Methanol = 9/1).

rote Kristalle, Fp: 115,5 - 17°C
Ausbeute: 3,8 g ( 21% d.Th.)
C ber. 51,79 % gef. 51,75 %
H ber. 6,52 % gef. 6,50 %
N ber. 30,20 % gef. 30,25 %

Beispiel 2

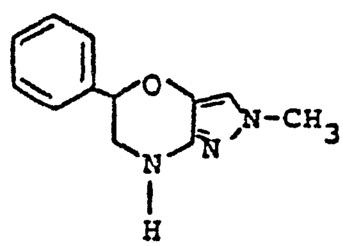

2-Methyl-5-phenylpyrazolo/4.3-b7/1.47oxazin

Zu einer Lösung von 9 ml (0,128 Mol) Dimethylformamid tropfte man unter Kühlung (5 bis 10°C) 11 ml (0,113 Mol) 2-Phenylmorpholon-5 in 25 ml abs. 1,2-Dichlorethan ein. Man ließ noch eine Stunde nachrühren und erwärmte dann zwei Stunden auf 70°C. Nach Abkühlen der Lösung schüttelte man sie mit 30 ml einer wäßrigen Natriumacetatlösung (12 g) aus. Die organische Phase wurde abgetrennt und mit wäßriger 0,5 molarer Natriumhydrogencarbonatlösung gewaschen, bis keine Gasentwicklung mehr auftrat. Nach Trocknen mit Natriumsulfat dampfte man i.V. das

Lösemittel ab (Rotavapor) und nahm den orangeroten, flüssigen Rückstand in 20 ml Ethanol auf. Hierzu tropfte man langsam bei Raumtemperatur 6,0 g (0,128 Mol) Hydrazinhydrat in 5 ml Ethanol und erwärmte anschließend noch eine Stunde am Rückfluß. Man destillierte den Alkohol i.V. ab (Rotavapor) und reinigte den Rückstand säulenchromatographisch (Kieselgel 60, Merck; Laufmittel: Dichlormethan/Methanol = 10/1). Rekristallisation erfolgte aus Dichlormethan/Diisopropylether.
farblose Kristalle, Fp: 146°C
Ausbeute: 1,9 g (17 % d. Th.)
C ber. 66,96 % gef. 67,05 %
H ber. 6,08 % gef. 5,94 %

Beispiel 3

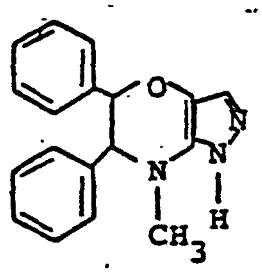

cis-7-Methyl-5,6-diphenylpyrazolo/4.3-b/ /1.4/oxazin

Man tropfte zu 15 ml Dimethylformamid (als Reaktionspartner und Lösemittel) langsam unter Rühren 3,5 g = 2,2 ml

(0,023 Mol) Phosphoroxychlorid. Dabei erwärmte sich die Lösung etwa handwarm. In diese Lösung gab man nach und nach 3,0 g (0,0112 Mol) cis-2,3-Diphenyl-4-methylmorpholon-5. Innerhalb von zwei Stunden ließ man unter Rühren auf Raumtemperatur abkühlen. Man rührte dreimal mit 20 ml Diethylether aus und dekantierte den Ether jeweils ab. Der Rückstand kristallisierte. Man nahm ihn in 50 ml Ethanol auf und tropfte unter Rühren und Kühlung 5,6 g (0,0112 Mol) Hydrazinhydrat in 50 ml Ethanol zu. Anschließend erhitzte man sechs Stunden zum Sieden. Beim Abkühlen fielen gelbe Kristalle aus; die Kristallisation wurde durch weiteres Abkühlen im Kühlschrank vervollständigt, Rekristallisation erfolgte aus Ethanol.

gelbliche Kristalle, Fp: 270°C (Zers.)
Ausbeute: 2,5 g (76 % d. Th.)
C ber. 74,20 % gef. 74,38 %
H ber. 5,88 % gef. 6,03 %

Beispiel 4

cis

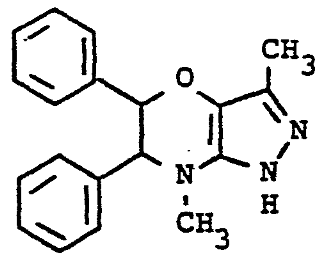

3,7-Dimethyl-5,6-diphenylpyrazolo/4.3-b7/1.47oxazin

54 g (0,2 Mol) 4-Methyl-2,3-diphenylmorpholon-5 und 98,7 g (0,64 Mol) Phosphoroxychlorid in 250 ml Ethylenchlorid werden zum Sieden erhitzt. Dabei verfärbt sich die Lösung braun bis dunkelbraun. Zur siedenden Lösung tropft man innerhalb von 2,5 Stunden 56,2 g (0,64 Mol) frisch dest. Dimethylacetamid. Anschließend dampft man das Lösemittel im Vakuum ab und nimmt den Rückstand unter Eiskühlung in 500 ml Ethanol auf. Man versetzt mit 300 ml Hydrazinhydrat (6,2 Mol) und erhitzt 18 Stunden zum Sieden. Nach Abdampfen des Lösemittels im Vakuum nimmt man den Rückstand in 1 l Wasser auf und extrahiert viermal mit 250 ml Dichlormethan. Man trocknet mit Natriumsulfat, dampft im Vakuum zur Trockne ein und nimmt den bräunlichen Rückstand unter gelindem Erwärmen in 250 ml Essigsäureethylester auf. Bei -18°C kristallisiert das gewünschte Produkt aus. Man saugt ab, wäscht mit Diisopropylether und Petrolether (40-60) und trocknet.
Farblose Kristalle, Fp.: 237-239°C (Z.)
Ausbeute: 25,9 g (42,4 % der Theorie)

Aus der Mutterlauge lassen sich noch zusätzlich 5,6 g (9,1 % der Theorie) gewinnen.
Gesamtausbeute demnach: 31,5 g (53,5 % der Theorie).

TP 49

Beispiel 5

cis

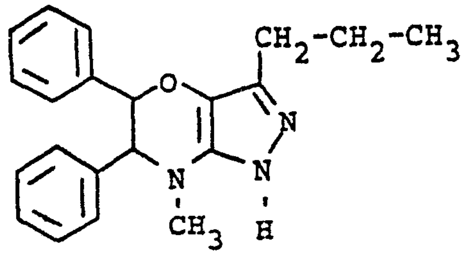

7-Methyl-3-propyl-5,6-diphenylpyrazolo/4.3-b7/1.47oxazin

8 g (0,03 Mol) 4-Methyl-2,3-diphenylmorpholon-5 und 13,8 g = 8,4 ml (0,09 Mol) Phosphoroxychlorid werden in 100 ml Ethylenchlorid auf 50°C erwärmt und bei dieser Temperatur mit 10,4 g (0,09 Mol) N,N-Dimethylbuttersäureamid in 100 ml Ethylenchlorid unter Rühren versetzt. Um die zu langsame Umsetzung zu beschleunigen (DC-Kontrolle), wird noch 4 Stunden zum Sieden erhitzt. Nach Abdampfen des Lösemittels im Vakuum nimmt man den trockenen Rückstand in 100 ml Ethanol auf und versetzt so langsam mit 51,6 g = 50 ml (1,03 Mol) Hydrazinhydrat, daß 5°C nicht überschritten werden. Anschließend erhitzt man noch 6 Stunden zum Sieden. Man dampft das Lösemittel im Vakuum ab, nimmt den Rückstand in 150 ml Wasser auf und extrahiert viermal mit ca. 70 ml Essigsäureethylester. Nach Trocknen mit Natriumsulfat wird im Vakuum auf ein kleines Volumen eingeengt und säulenchromatographiert (Kieselgel 60, Laufmittel:Dichlormethan:Methanol = 20:1 bis 10:1).

Farblose Kristalle, Fp.: 164°C

Ausbeute: 4,0 g (40 % der Theorie).

Beispiel 6

cis

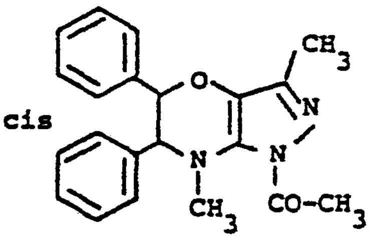

1-Acetyl-3,7-dimethyl-5,6-diphenylpyrazolo[4.3-b][1.4]-oxazin

2,2 g (0,0072 Mol) 3,7-Dimethyl-5,6-diphenylpyrazolo-[4.3-b][1.4]oxazin werden in 50 ml Diethylether suspendiert, mit 2,4 ml = 2,3 g (0,029 Mol) Pyridin versetzt und auf 0°C abgekühlt. Bei 0 bis 5°C wird zu dieser Suspension unter Rühren eine Lösung von 1 ml = 1,1 g (0,014 Mol) Acetylchlorid in 10 ml Diethylether getropft. Man läßt noch 1 Stunde rühren und auf Raumtemperatur erwärmen. DC-Kontrolle zeigt vollständige Umsetzung an. Es wird auf Eis geschüttet und dreimal mit je ca. 50 ml Dichlormethan extrahiert. Nach Trocknen mit Natriumsulfat und Einengen auf ein kleines Volumen wird säulenchromatographisch gereinigt (Kieselgel 60, Laufmittel: Dichlormethan/Methanol = 10/1).
Farblose Kristalle, Fp.: 160,2°C
Ausbeute: 1,6 g (64,0 % der Theorie).

Analog diesen Beispielen wurden die folgenden erfindungsgemäßen Verbindungen hergestellt:

Tabelle 1

| Nr. | Struktur | Fp [°C] | Ausbeute [% d. Th.] |
|---|---|---|---|
| 1 | 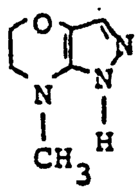 | 117 | 21 |
| 2 | 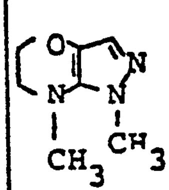 + Isom. | ca. 46 | 25 |
| 3 | 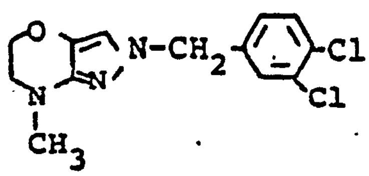 | 69 | 15 |
| 4 | 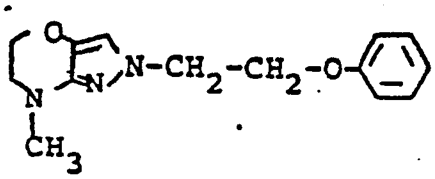 | 71 | 7 |

Tabelle 1 (Fortsetzung)

| Nr. | Struktur | Fp [°C] | Ausbeute [% d. Th.] |
|---|---|---|---|
| 5 | 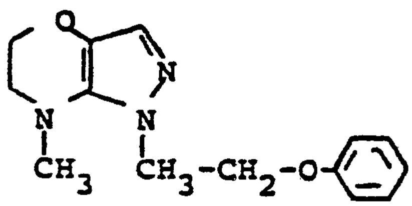 + Isom. | ca. 67 | 7 |
| 6 | 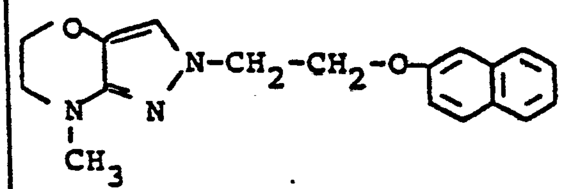 | 65 | 20 |
| 7 | 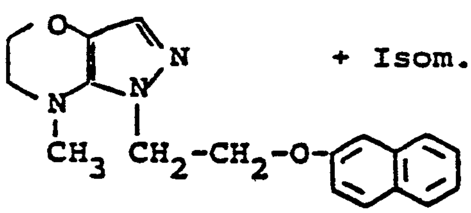 + Isom. | ca. 60 | 8 |
| 8 | 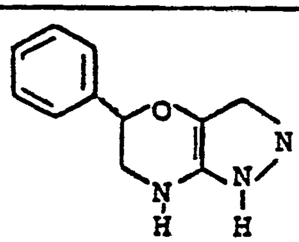 | 171 | 11 |

Tabelle 1 (Fortsetzung)

| Nr. | Struktur | Fp [°C] | Ausbeute [% d. Th.] |
|---|---|---|---|
| 9 | 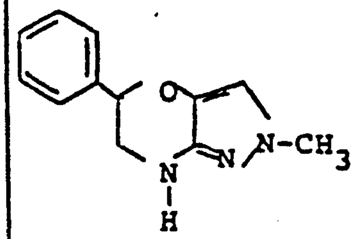 | 148 | 17 |
| 10 | 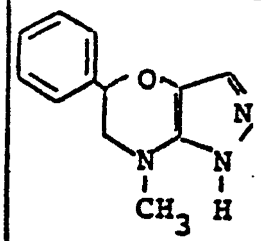 | 173 | 75 |
| 11 | 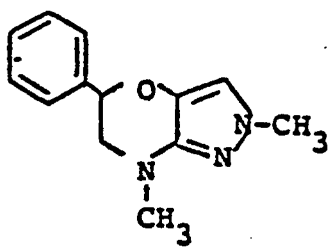 | 85 | 63 |
| 12 | 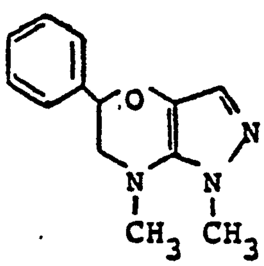 | 107 | 5 |

Tabelle 1 (Fortsetzung)

| Nr. | Struktur | Fp [°C] | Ausbeute [% d. Th.] |
|---|---|---|---|
| 13 | 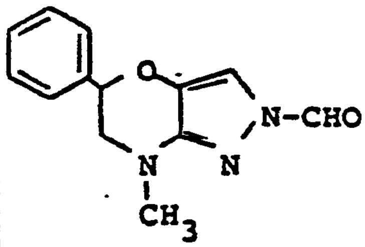 | 131 | 80 |
| 14 | 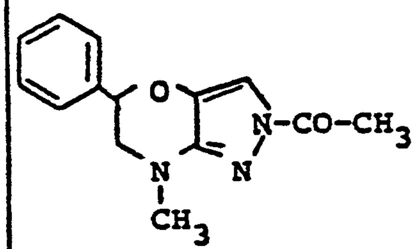 | 101 | 84 |
| 15 | 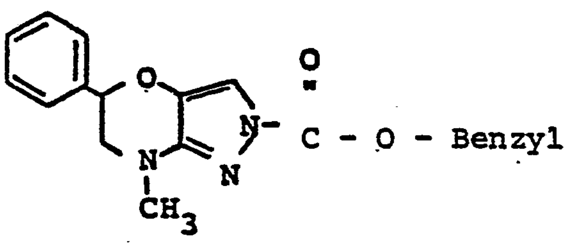 | 147 | 77<br>Benzyl |

Tabelle 1 (Fortsetzung)

| Nr. | Struktur | Fp [°C] | Ausbeute [% d. Th.] |
|---|---|---|---|
| 16 | 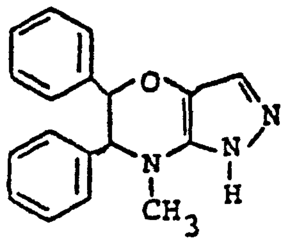 cis | 270 Z | 76 |
| 17 | 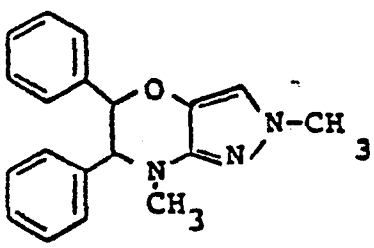 cis | 115 | 60 |
| 18 | 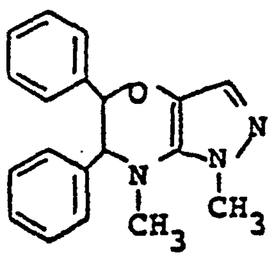 cis | 158 | 9 |
| 19 | 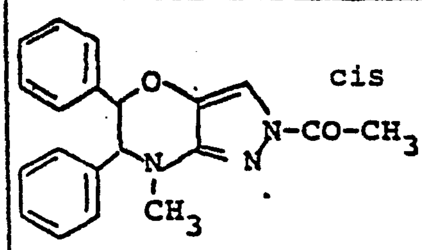 cis | 135 | 83 |
| 20 | 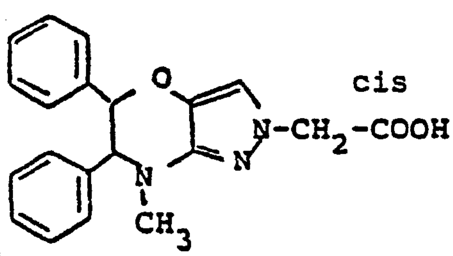 cis | 201 Z | 63 |

Tabelle 1 (Fortsetzung)

| Nr. | Struktur | | Fp [°C] | Ausbeute [% d. Th.] |
|---|---|---|---|---|
| 21 | 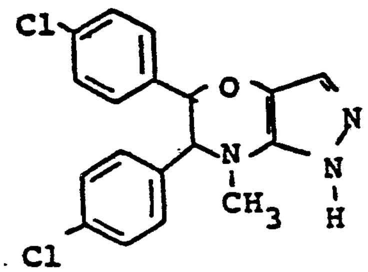 | cis | 269 Z | 87 |
| 22 | 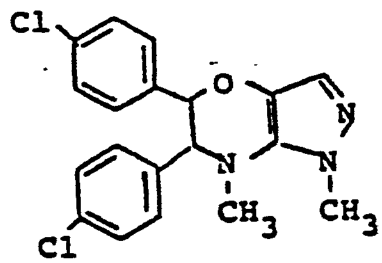 | cis | 192 Z | 53 |
| 23 | 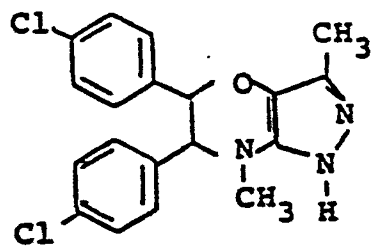 | cis | 204 Z | 39 |
| 24 | 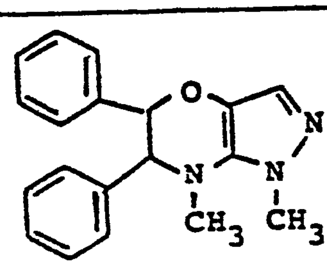 | trans | 156 | 73 |

Tabelle 1 (Fortsetzung)

| Nr. | Struktur | | Fp [°C] | Ausbeute [% d. Th.] |
|---|---|---|---|---|
| 25 | 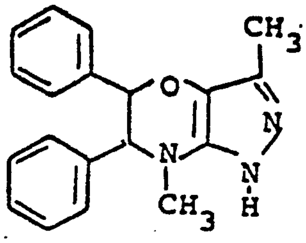 | cis | 239 Z | 15 |
| 26 | 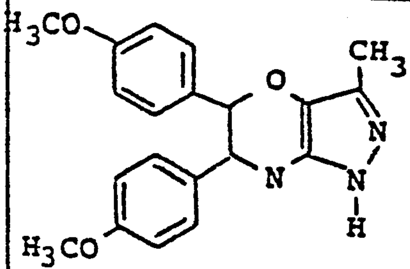 | x HCl cis | 249 Z | 25 |
| 27 | 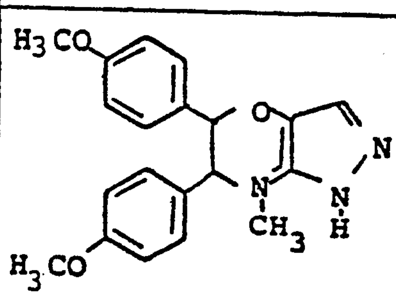 | cis | 246 Z | 76 |
| 28 | 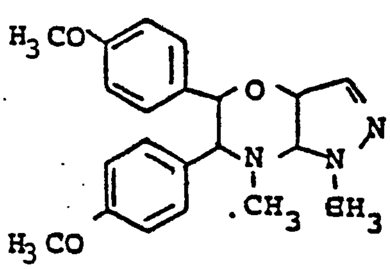 | cis | 115 | 37 |

In Analogie zu Beispiel 4 wurden die folgenden Verbindungen der Tabelle 1 erhalten:

Tabelle 2

| Nr. | Struktur | Fp (°C) | Ausbeute (% d.Th.) |
|---|---|---|---|
| 1 | 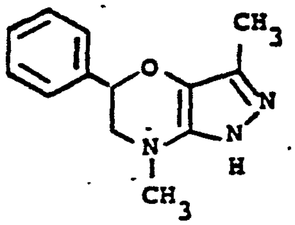 | 163 | 19 |
| 2 | 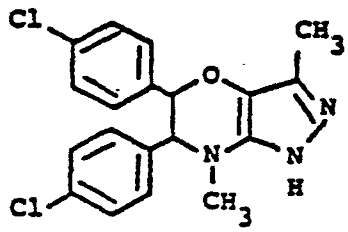 | 204 (Zers.) | 39 |
| 3 | 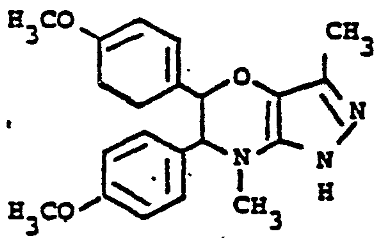 . HCl | 239-240 (Zers.) | 25 |
| 4 | 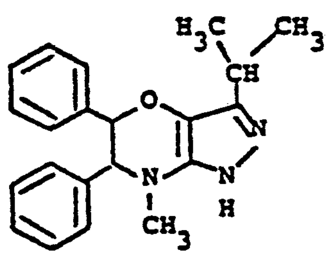 | 183 | 17 |

Patentansprüche

1. Verbindungen der allgemeinen Formel I in ihren isomeren Formen Ia und Ib, sowohl einzeln als auch im Gemisch,

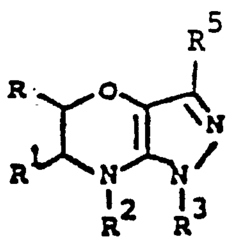

Ia

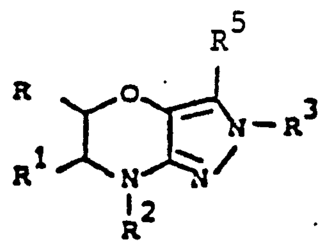

Ib

I

wobei

R und $R^1$ gleich oder verschieden sein können und für Wasserstoff, Alkyl mit 1 bis 18 C-Atomen und Phenyl, gegebenenfalls substituiert mit bis zu fünf gleichen oder verschiedenen Substituenten aus der Gruppe Alkyl mit 1 bis 6 C-Atomen, Cycloalkyl mit 3 bis 6 C-Atomen, Aralkyl mit 7 bis 22 C-Atomen, Aryl mit 6 bis 14 C-Atomen, Mono- und Dialkylamino mit 1 bis 12 C-Atomen, Arylamino mit 6 bis 10 C-Atomen, Aryloxy mit 6 bis 10 C-Atomen, Alkylthio mit 1 bis 12 C-Atomen, Arylthio mit 6 bis 10 C-Atomen, Aralkoxy mit 7 bis 22 C-Atomen, Aralkylamino mit 7 bis 16 C-Atomen, Carboxy, Carbalkoxy mit 2 bis 13 C-Atomen,

Carbamoyl, Mono- und Dialkylcarbamoyl mit 1 bis 12 C-Atomen, Alkylsulfonyl mit 1 bis 12 C-Atomen, Arylsulfonyl mit 6 bis 14 C-Atomen, Halogenalkyl mit bis zu 12 C-Atomen, ggf. einfach oder mehrfach substituiert mit F, Cl, Br oder/und J, Hydroxyalkyl mit 1 bis 12 C-Atomen, Aminoalkyl mit 1 bis 12 C-Atomen, Alkoxycarbonylaminoalkyl mit 3 bis 13 C-Atomen, Halogenalkoxy mit bis zu 12 C-Atomen, ggf. einfach oder mehrfach substituiert mit F, Cl, Br oder/und J, Halogen wie F, Cl, Br und J, Amino, Acylamino mit 2 bis 7 C-Atomen, Carbamoylamino, Sulfonylamino, Hydroxy, Acyloxy mit 2 bis 7 C-Atomen, Sulfonamido, Nitro, Formyl, Alkylcarbonyl mit 2 bis 13 C-Atomen, Arylcarbonyl mit 7 bis 15 C-Atomen, Methylencarboxy, Methylencarboxyalkyl mit 3 bis 8 C-Atomen, Ethylencarboxyalkyl mit 4 bis 9 C-Atomen oder Propylencarboxyalkyl mit 5 bis 10 C-Atomen stehen,

$R^2$ und $R^3$ gleich oder verschieden sein können und für Wasserstoff, Alkyl mit 1 bis 18 C-Atomen stehen, wobei der Alkylrest durch Sauerstoff, Schwefel oder Stickstoff ein- oder mehrfach unterbrochen sein kann, Alkenyl oder Alkinyl mit 2 bis 12 C-Atomen, Aralkyl mit 7 bis 12 C-Atomen, wobei die Alkenyl-, Alkinyl- und Aralkylreste ebenfalls durch Sauerstoff, Schwefel oder Stickstoff ein- oder mehrfach unterbrochen sein können, für Aryl stehen mit 6 bis 14 C-Atomen, 5- bis 6-gliedriges Heteroaryl mit bis zu 4

Heteroatome aus der Reihe N, O und S, 5- bis 6-gliedriges Heteroarylalkyl mit bis zu 4 Heteroatomen aus der Reihe N, O und S mit 1 bis 12 C-Atomen in der Alkylkette, Alkylcarbonyl mit 2 bis 13 C-Atomen, Arylcarbonyl mit 7 bis 11 C-Atomen, ggf. mit bis zu 5 gleichen oder verschiedenen Substituenten aus der Gruppe Alkyl mit 1 bis 18 C-Atomen, Cycloalkyl mit 3 bis 6 C-Atomen, Aralkyl mit 7 bis 22 C-Atomen, Aryl mit 6 bis 14 C-Atomen, Mono- und Dialkylamino mit 1 bis 12 C-Atomen, Arylamino mit 6 bis 10 C-Atomen, Aryloxy mit 6 bis 10 C-Atomen, Alkylthio mit 1 bis 12 C-Atomen, Arylthio mit 6 bis 10 C-Atomen, Aralkoxy mit 7 bis 22 C-Atomen, Aralkylamino mit 7 bis 16 C-Atomen, Carboxy, Carbalkoxy mit 2 bis 13 C-Atomen, Carbamoyl, Mono- und Dialkylcarbamoyl mit 1 bis 12 C-Atomen, Alkylsulfonyl mit 1 bis 12 C-Atomen, Arylsulfonyl mit 6 bis 14 C-Atomen, Halogenalkyl mit bis zu 12 C-Atomen, ggf. einfach oder mehrfach substituiert mit F, Cl, Br oder/und J, Hydroxyalkyl mit 1 bis 12 C-Atomen, Aminoalkyl mit 1 bis 12 C-Atomen, Alkoxycarbonylaminoalkoxy mit 3 bis 13 C-Atomen, Halogenalkoxy mit bis zu 12 C-Atomen, ggf. einfach oder mehrfach substituiert mit F, Cl, Br oder/und J, Halogen wie F, Cl, Br oder/und J, Amino, Acylamino mit 2 bis 7 C-Atomen, Sulfonamido, Nitro, Formyl, Alkylcarbonyl mit 2 bis 13 C-Atomen, Arylcarbonyl mit 7 bis 15 C-Atomen, Methylencarboxy, Methylencarboxyalkyl mit 3

bis 8 C-Atomen, Ethylencarboxyalkyl mit 4 bis 9 C-Atomen oder Propylencarboxyalkyl und für 5- bis 6-gliedriges Heterarylcarbonyl mit bis zu 4 Heteroatomen aus der Reihe N, O und S und

$R^5$ für Wasserstoff, für einen unverzweigten, verzweigten oder cyclischen Alkylrest steht, jeweils mit 3 bis 6 C-Atomen, welcher ggf. durch Hydroxy, Carboxy, Formyl, Acetyl, Alkoxy, Alkylthio, Halogen, Nitro, substituierte oder freie Aminogruppen substituiert sein kann, für einen aromatischen oder heteroaromatischen Rest mit bis zu 4 Heteroatomen aus der Reihe N, O und S steht, der ggf. durch Alkyl, Alkoxy, Alkylthio oder Halogen substituiert sein kann oder für einen in gleicher Weise substituierten Aralkyl- oder Heteroaralkylrest steht.

2. Verbindungen der allgemeinen Formel Ia und Ib gemäß Anspruch 1, in welcher

R und $R^1$ gleich oder verschieden sein können und für Wasserstoff, Alkyl mit 1 bis 6 C-Atomen und Phenyl, ggf. mit bis zu 5 gleichen oder verschiedenen Substituenten aus der Gruppe Alkyl mit 1 bis 6 C-Atomen, Aralkyl mit 7 bis 15 C-Atomen, Phenyl oder Naphthyl, Mono- und Dialkylamino mit 1 bis 8 C-Atomen, Arylamino mit 6 bis 10 C-Atomen, Aryloxy mit 6 bis 10 C-Atomen, Alkylthio mit 1 bis 12 C-Atomen, Aralkoxy mit 7 bis 16 C-Atomen, Aralkylamino mit 7 bis 16 C-Atomen, Carboxy, Carbalkoxy mit 2 bis 7 C-Atomen,

Carbamoyl, Mono- und Dialkylcarbamoyl mit 1 bis 6 C-Atomen, Alkylsulfonyl mit 1 bis 12 C-Atomen, Arylsulfonyl mit 6 bis 14 C-Atomen, Halogenalkyl mit bis zu 6 C-Atomen, ggf. substituiert mit F, Cl, oder/und Br, Hydroxyalkyl mit 1 bis 6 C-Atomen, Aminoalkyl mit 1 bis 6 C-Atomen, Aminoalkoxycarbonylaminoalkoxy mit 3 bis 9 C-Atomen, Halogenalkoxy mit bis zu 6 C-Atomen, ggf. substituiert mit F, Cl oder/und Br, Halogen wie F, Cl und Br, Amino, Acylamino mit 2 bis 7 C-Atomen, Carbonylamino, Sulfonylamino, Hydroxy, Acyloxy mit 2 bis 7 C-Atomen, Sulfonamido, Nitro, Formyl, Alkylcarbonyl mit 2 bis 7 C-Atomen, Arylcarbonyl mit 7 bis 11 C-Atomen, Methylencarboxy, Methylencarboxyalkyl mit 3 bis 8 C-Atomen, Ethylencarboxyalkyl mit 4 bis 9 C-Atomen und Propylencarboxyalkyl substituiert stehen,

$R^2$ und $R^3$ gleich oder verschieden sein können und für Wasserstoff, Alkyl mit 1 bis 12 C-Atomen stehen, wobei der Alkylrest durch Sauerstoff, Schwefel oder Stickstoff ein- oder mehrfach unterbrochen sein kann, Alkenyl oder Alkinyl mit 2 bis 6 C-Atomen, Aralkyl mit 7 bis 12 C-Atomen, wobei die Alkenyl-, Alkinyl- und Aralkylrest ebenfalls durch Sauerstoff, Schwefel oder Stickstoff ein- oder mehrmals unterbrochen sein können, für Phenyl und Naphthyl stehen, 5- bis 6-gliedriges Heteroaryl mit bis zu drei Heteroatomen aus der Reihe N, O und S

5- bis 6-gliedriges Heteroarylalkyl mit bis zu drei.Heteroatomen aus der Reihe N, O und S mit 1 bis 6 C-Atomen in der Alkylkette, Alkylcarbonyl mit 2 bis 13 C-Atomen, Arylcarbonyl mit 7 bis 11 C-Atomen, ggf. mit bis zu 5 gleichen oder verschiedenen Substituenten aus der Gruppe Alkyl mit 1 bis 6 C-Atomen, Cyclalkyl mit 3 bis 6 C-Atomen, Aralkyl mit 7 bis 11 C-Atomen, Phenyl und Naphthyl, Mono- und Dialkylamino mit 1 bis 8 C-Atomen, Arylamino mit 6 bis 10 C-Atomen, Aryloxy mit 6 bis 10 C-Atomen, Alkylthio mit 1 bis 6 C-Atomen, Arylthio mit 6 bis 10 C-Atomen, Aralkoxy mit 7 bis 12 C-Atomen, Aralkylamino mit 7 bis 12 C-Atomen, Carboxy, Carbalkoxy mit 2 bis 7 C-Atomen, Carbamoyl, Mono- und Dialkylcarbamoyl mit 1 bis 6 C-Atomen, Alkylcarbonyl mit 1 bis 12 C-Atomen, Alkylsulfonyl mit 1 bis 12 C-Atomen, Arylsulfonyl mit 6 bis 14 C-Atomen, Halogenalkyl mit bis zu 6 C-Atomen, ggf. einfach oder mehrfach substituiert mit F, Cl oder/und Br, Hydroxyalkyl mit 1 bis 6 C-Atomen, Aminoalkyl mit 1 bis 6 C-Atomen, Alkoxycarbonylaminoalkoxy mit 3 bis 9 C-Atomen, Halogenalkoxy mit bis zu 6 C-Atomen, ggf. einfach oder mehrfach substituiert mit F, Cl oder/und Br, Halogen wie F, Cl oder/ und Br, Amino, Acylamino mit 2 bis 7 C-Atomen, Carbamoylamino, Sulfonylamino, Hydroxy, Acyloxy mit 2 bis 7 C-Atomen, Sulfonamido, Nitro, Formyl, Alkylcarbonyl mit 2 bis 13 C-Atomen, Arylcarbonyl mit 7 bis 15 C-Atomen, Methylencarboxy, Methylencarboxyalkyl mit 3 bis 8 C-Atomen, Ethylencarboxyalkyl mit 4 bis 9 C-Atomen oder Propylencarboxyalkyl mit 5 bis 10 C-Atomen und 5- bis 6-gliedriges Heteroarylcarbonyl mit bis zu drei Heteroatomen aus der Reihe N, O und S substituiert sein kann und

$R^5$ für Wasserstoff, für einen unverzweigten, verzweigten oder cyclischen Alkylrest steht, jeweils mit 3 bis 6 C-Atomen, welcher ggf. durch Hydroxy, Carboxy, Formyl, Acetyl, Alkoxy, Alkylthio, Halogen, Nitro, substituiertes oder freie Aminogruppen substituiert sein kann, für einen aromatischen oder heteroaromatischen Rest mit bis zu 4 Heteroatomen aus der Reihe N, O und S steht, der ggf. durch Alkyl, Alkoxy, Alkylthio oder Halogen substituiert sein kann oder für einen in gleicher Weise substituierten Aralkyl- oder Heteroaralkylrest steht.

3. Verbindungen der allgemeinen Formeln Ia und Ib gemäß Anspruch 1 oder 2, in welcher

R und $R^1$ gleich oder verschieden sein können und für Wasserstoff, Alkyl mit 1 bis 6 C-Atomen, ggf. mit bis zu 5 Fluoratomen substituiert und/oder Phenyl stehen, ggf. mit bis zu 5 Substituenten substituiert aus der Gruppe Alkyl mit 1 bis 4 C-Atomen je Alkylgruppe, Alkyloxy mit 1 bis 3 C-Atomen, Aryloxy mit 6 C-Atomen und mit F, Cl, Br, $NO_2$, $NH_2$, NH-niederes Alkyl, N(niederes Alkyl)$_2$, Alkylthio mit 1 bis 6 C-Atomen, Alkylsulfonyl mit 1 bis 6 C-Atomen, mit Aminocarbonyl und Aminosulfonyl,

$R^2$ und $R^3$ gleich oder verschieden sind und für Wasserstoff, Alkyl mit bis zu 6 C-Atomen, Arylalkyl mit 3 bis 13 C-Atomen, ggf. substituiert mit bis zu 5 F, Cl, oder Br, Aryloxyalkyl mit 7 bis 16 C-Atomen, Formyl, Alkylcarbonyl mit 2 bis 13 C-Atomen, Carboxyalkyl mit 2 bis 7 C-Atomen und für Arylalkyloxycarbonyl mit 8 bis 16 C-Atomen stehen und

$R^5$ für Wasserstoff, für einen unverzweigten oder verzweigten Alkylrest mit 1 bis 6 C-Atomen steht, der ggf. durch Alkoxy substituiert sein kann, oder für einen Aryl- oder Heteroarylrest steht; bevorzugt sind Phenyl- oder Pyridylreste, die ggf. durch Fluor, Chlor, Alkyl oder Alkoxy substituiert sind.

4. Verbindungen der allgemeinen Formel I in ihren isomeren Formen Ia und Ib,

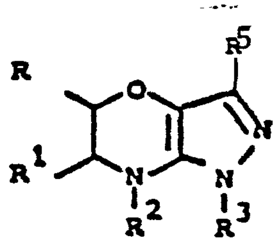

Ia

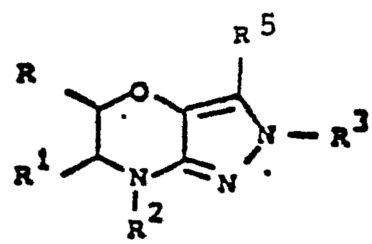

Ib

worin
R und $R^1$ für Wasserstoff, Methyl und/oder Phenyl, 4-Chlorphenyl, 4-Methoxyphenyl stehen,
$R^2$ Wasserstoff, Methyl oder Ethyl bedeutet und

$R^3$ für Wasserstoff, Methyl, 3,4-Dichlorbenzyl, 2-Phenoxyethyl, ß-Naphthoxyethyl, Benzyloxycarbonyl, Formyl, Acetyl und Carboxymethyl steht und

$R^5$ für Wasserstoff, für Methyl, Propyl und Isopropyl steht.

5. Verbindungen der allgemeinen Formel Ia und Ib gemäß einem oder mehreren der Ansprüche 1 bis 4 zur Verwendung bei der Behandlung von Krankheiten.

6. Verfahren zur Herstellung von Verbindungen der allgemeinen Formeln Ia und Ib gemäß Ansprüchen 1 bis 4, dadurch gekennzeichnet, daß man Verbindungen der allgemeinen Formel II

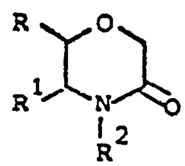

II

in welcher

R, $R^1$ und $R^2$ die im Anspruch 1 angegebene Bedeutung besitzen,

mit einem N,N-Dialkylamid der allgemeinen Formel VI mit der im Anspruch 1 angegebenen Bedeutung und Phosphoroxychlorid umsetzt,

und die dabei entstehenden Salze mit Hydrazinen der allgemeinen Formel

$$R^3 - NH - NH_2,$$

worin

$R^3$ Wasserstoff, Alkyl mit 1 bis 18 C-Atomen, Aralkyl mit 7 bis 12 C-Atomen, Heteroalkyl, wobei die Alkyl-, Aralkyl- und Heteroaralkylreste durch Sauerstoff, Schwefel und Stickstoff ein- oder

mehrmals unterbrochen sein können, Aryl mit 6 bis 10 C-Atomen und Heteroaryl bedeutet, wobei die Aryl- und Heteroarylreste mit bis zu 5 gleichen oder verschiedenen Substituenten aus der Gruppe Alkoxy, Alkyl, Aralkyl, Cycloalkyl, Aryl, Alkylamino, Dialkylamino, Arylamino, Aryloxy, Arylthio, Alkylthio, Carboxy, Carbalkoxy, Cyano, Carbamoyl, Sulfonyl, Alkylsulfonyl, Halogenalkoxy, Halogen, Amino oder substituiertes Amino, Sulfonamido, Methylencarboxy, Methylencarboxyalkyl oder Propylencarboxyalkyl substituiert sein kann,

bei Temperaturen zwischen - 20 °C und + 100 °C umsetzt, oder die Oxazine II (wie oben) mit Carbonsäureestern VII mit der im Anspruch I angegebenen Bedeutung acyliert, die entstandenen Acylprodukte VIII nach Anspruch 1 schwefelt und die entstandenen Thione IX mit Hydrazinen der allgemeinen Formel IV (wie oben) bei Temperaturen zwischen - 20 °C und + 100 °C umsetzt.

7. Verwendung von Verbindungen der allgemeinen Formel I gemäß Ansprüchen 1 bis 4 bei der Bekämpfung von Krankheiten, bevorzugt von entzündlichen Prozessen, insbesondere als Lipoxygenasehemmer und/oder als Analgetika als Gefäßwand- und/oder Cytoprotektiva, besonders als Antithrombotikum, Antimetastatikum, Antiallergikum, Antiasthmatikum, Ulcus-Präventivum und/oder Anurektikum.

8. Arzneimittel, enthaltend mindestens eine Verbindug der allgemeinen Formeln gemäß Ansprüchen 1 bis 4.

9. Arzneimittel nach Anspruch 8, dadurch gekennzeichnet, daß es eine oder mehrere Wirksubstanzen in einer Menge von 0,5 bis 90 Gew.-% der Gesamtformulierung enthält.

10. Verfahren zur Herstellung von Arzneimitteln, dadurch gekennzeichnet, daß man Verbindungen der allgemeinen Formeln Ia und Ib gemäß Ansprüchen 1 bis 3, gegebenenfalls unter Verwendung von üblichen Hilfs- und Trägerstoffen in eine geeignete Applikationsform überführt.

0111809
Nummer der Anmeldung

Europäisches Patentamt

**EUROPÄISCHER TEILRECHERCHENBERICHT,**
der nach Regel 45 des Europäischen Patentübereinkommens für das weitere Verfahren als europäischer Recherchenbericht gilt

EP 83112196.7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| P,X | EP - A2 - 0 085 881 (BAYER)<br>* Gesamt *<br>-- | 1-6, 8-10 | C 07 D 498/04<br>C 07 D 265/28<br>A 61 K 31/535 |
| Y | US - A - 4 276 292 (GEORGIEV et al.)<br>* Spalten 1,2; Patentansprüche *<br>-- | 1-5, 8-10 | |
| Y | DD - A - 148 778 (PESEKE, KOLLHOFF)<br>* Erfindungsanspruch *<br>-- | 1,6 | |
| A | AT - B - 265 301 (MC NEIL LAB.)<br>* Patentanspruch *<br>-- | 6 | |

RECHERCHIERTE SACHGEBIETE (Int. Cl. 3)

C 07 D 498/00
C 07 D 265/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-6,8-10
Unvollständig recherchierte Patentansprüche: –
Nicht recherchierte Patentansprüche: 7
Grund für die Beschränkung der Recherche:

Verfahren zur therapeutischen Behandlung des menschlichen oder tierischen Körpers (siehe Art. 52(4) des Europäischen Patentübereinkommens)

| Recherchenort | Abschlußdatum der Recherche | Prüfer |
|---|---|---|
| WIEN | 24-02-1984 | JANISCH |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03 82

0111809

Nummer der Anmeldung

Europäisches Patentamt

# EUROPÄISCHER TEILRECHERCHENBERICHT

EP 83112196.7

| EINSCHLÄGIGE DOKUMENTE | | | KLASSIFIKATION DER ANMELDUNG (Int. Cl.3) |
|---|---|---|---|
| Kategorie | Kennzeichnung des Dokuments mit Angabe, soweit erforderlich, der maßgeblichen Teile | betrifft Anspruch | |
| D,A | TETRAHEDRON, Vol. 25, 1969, Pergamon Press,<br>M. MAZHARUDDIN & G. THYAGARAJAN, "Studies on 1,4-Benzoxazines-1..." Seiten 517-525<br>* Seiten 518,522, letzter Absatz, 524(XIII,XIV) *<br>---- | 6 | |
| | | | RECHERCHIERTE SACHGEBIETE (Int. Cl.3) |